Europäisches Patentamt

European Patent Office

Office européen des brevets

(19)

(11) Numéro de publication : **0 487 435 A2**

# (12) DEMANDE DE BREVET EUROPEEN

(21) Numéro de dépôt : **91430025.6**

(22) Date de dépôt : **20.11.91**

(51) Int. Cl.$^5$ : **A61B 17/36,** A61C 1/00, B23K 26/06

(30) Priorité : **20.11.90 US 615789**

(43) Date de publication de la demande : **27.05.92 Bulletin 92/22**

(84) Etats contractants désignés :
**AT BE CH DE DK ES FR GB GR IT LI LU NL SE**

(71) Demandeur : **Levy, Guy
2874 Calle Belmonte
Tustin, California (US)**

(72) Inventeur : **Levy, Guy
2874 Calle Belmonte
Tustin, California (US)**

(74) Mandataire : **Marek, Pierre
28 & 32 rue de la Loge
F-13002 Marseille (FR)**

(54) **Procédé et appareil utilisant la radiation laser pour interventions sur des tissus minéralisés divers.**

(57) Appareil utilisant la radiation laser pour interventions sur des tissus minéralisés divers, tels que, par exemple, des tissus physiologiques minéralisés incluant, en particulier, l'os et les différents tissus dentaires, cet appareil comprenant une source d'émission (2) d'un rayonnement laser et une fibre optique (8) ayant une extrémité proximale (8a) disposée pour recevoir le rayonnement émis par la source laser (2) et une extrémité distale (8b) dimensionnée pour pouvoir être introduite dans un canal dentaire ou autre, caractérisé en ce qu'un dispositif (6) de polarisation linéaire est intercalé entre ladite source laser (2) et ladite extrémité proximale (8a) de la fibre optique (8), ce dispositif (6) permettant une polarisation linéaire du faisceau ou rayonnement émis par la source laser.

Fig. 1

EP 0 487 435 A2

La présente invention concerne un procédé utilisant la radiation laser pour interventions sur des tissus minéralisés divers tels que, par exemple des tissus physiologiques minéralisés incluant, en particulier, l'os et les différents tissus dentaires.

Une application précédente du rayonnement laser décrite dans les documents EP-A-0.375.578 et EP-A-0.392.951, concernait des appareils et procédés pour couper ou vaporiser les tissus physiologiques minéralisés par exemple l'émail, la dentine, le cément et l'os. Il a été démontré, dans ces applications, que ces interventions pouvaient être réalisées par des émissions à des longueurs d'ondes qui sont absorbées très largement par l'hydroxyapatite qui est un constituant principal de ces tissus physiologiques et seulement absorbée très faiblement par l'eau. Une source laser plus particulièrement indiquée pour ces interventions, est le ND-YAG dont l'émission ou longueur d'onde normale de 1,06 μm est plus largement absorbée par l'hydroxyapatite que par l'eau.

Dans les documents susmentionnés, a également été décrite, la possibilité de réaliser des élargissements ou autres traitements dans les canaux des racines dentaires, à l'aide de fibres optiques introduites dans ces canaux ou passages. Il a été démontré que la fibre peut effectuer une coupe des racines dentaires ou différents traitements à l'apex de la racine tels qu'apicoectomie ou amputation.

Des techniques pour remplir des cavités ou autres ouvertures dans la dent et l'os ont également été décrites dans ces documents antérieurs.

Les travaux de recherches et d'études ultérieurs ont permis de découvrir d'importantes améliorations aux procédures et matériels décrits dans les documents précités et ces perfectionnements font l'objet de l'invention exposée ci-après.

Un premier objet de l'invention est d'améliorer l'efficacité de la coupe ou de la vaporisation des tissus précédemment cités, au moyen d'un rayonnement laser.

Un autre objet de la présente invention est d'améliorer l'élargissement des canaux et passages à l'aide de l'émission laser.

Un autre objet de l'invention est encore d'améliorer le remplissage ou le recouvrement de ces passages, cavités, fentes ou fissures, tels que le canal d'une dent ou un canal dans l'os, l'ivoire ou objets divers réalisés à l'aide de ces produits.

Un autre objet de l'invention est de permettre aussi des opérations sur des objets métalliques situés ou non dans la bouche.

Les buts ci-dessus et d'autres objectifs, sont atteints, selon une disposition caractéritique de l'invention, par un procédé de coupe des tissus physiologiques minéralisés de la dent, c'est-à-dire l'émail, l'ivoire, la dentine ou l'os, ou d'autres matières ou tissus minéralisés non physiologiques, ce procédé comprenant : l'émission d'une radiation ou rayonnement laser à une longueur d'onde qui est absorbée plus largement par l'hydroxyapatite que par l'eau, cette émission ou rayonnement étant polarisée linéairement et focalisée pour obtenir un rayon de faible diamètre, le rayon polarisé étant appliqué sur la surface du tissu à couper de façon que ledit rayon polarisé puisse avoir un angle d'incidence sélectionné par rapport à ladite surface à couper ; de la sorte, l'émission ou rayonnement laser appliquée sur la surface, a une polarisation P parallèle à celle-ci.

L'invention concerne aussi un dispositif ou appareil utilisant la radiation laser et permettant d'effectuer diverses interventions sur des tissus minéralisés, cet appareil pouvant par exemple être destiné à couper ou à vaporiser des tissus ou substances calcifiés ou minéralisés, tels que les tissus physiologiques des canaux dentaires. Cet appareil comprend une source de rayonnement Laser et une fibre optique ayant une extrémité proximale disposée pour recevoir l'émission ou rayonnement provenant de la source laser et une extrémité distale dimensionnée pour pouvoir être introduite dans les canaux dentaires, cette fibre optique ayant une forme effilée dans sa partie incluant son extrémité distale, de sorte que le diamètre de la fibre décroît en direction de ladite extrémité distale.

Un objet supplémentaire de la présente invention, est constitué par un procédé pour remplir ou recouvrir des matériaux calcifiés ou des tissus minéralisés tels que, par exemple, les tissus physiologiques de la dent : émail, dentine, ivoire, ou l'os, avec un produit contenant du calcium et apte à être fixé ou soudé à la surface desdits tissus, ce remplissage ou ce recouvrement étant réalisés au moyen d'au moins deux composants initiaux qui, lorsqu'ils sont mélangés et exposés à un rayonnement présentant une longueur d'onde convenable, produisent une réaction photo-chimique aboutissant à la formation dudit produit calcique, ce procédé comprenant la projection des composants sur la surface des tissus et l'application d'un rayonnement laser de la longueur d'onde sélectionnée sur ces composants, de façon que ceux-ci soient atteints et irradiés, au moins partiellement, avant qu'ils n'atteignent ladite surface, de sorte à produire la réaction photo-chmique permettant au produit calcique obtenu de se fixer sur les tissus ou matières minéralisés.

Suivant une autre disposition caractéristique du procédé de l'invention appliquée au remplissage ou au recouvrement d'un passage ou d'un canal, on utilise une fibre optique contenant de la silice ayant un point de fusion bas et dimensionnée pour pouvoir être introduite dans les canaux, on engage cette fibre à l'intérieur du canal de façon qu'elle s'étende le long de la zone à remplir ou à recouvrir, et on injecte dans la fibre optique, un rayon laser de longueur d'onde et de niveau d'énergie suffisants pour fondre la portion de fibre insérée dans le canal et permettre à la silice de se fixer sur les parois canalaires environnantes.

Un autre objet du procédé de l'invention consiste à détruire des bactéries sur la surface des dents ou autres surfaces de tissus minéralisés, en colorant les bactéries avec un colorant sombre ou noir, et à exposer ensuite ces bactéries colorées à un rayonnement laser, à une longueur d'onde et à un niveau d'énergie suffisants pour permettre leur destruction.

Selon une autre disposition caractéristique de l'invention, le type de rayonnement laser décrit peut être utilisé pour mettre en fusion, c'est-à-dire fondre, ou souder, ou vaporiser, des objets métalliques fixés à l'os ou aux tissus de la dent, y compris les tissus de la gencive, ou logés dans les canaux dentaires. Ceci permet d'installer, d'enlever ou d'adapter des implants métalliques et de vaporiser des instruments tels que des pointes de limes brisées, bloquées dans un canal. Ces interventions peuvent s'effectuer conjointement avec une pulvérisation ou écoulement d'un liquide de refroidissement pour éviter la brûlure ou la carbonisation des tissus voisins.

Suivant une autre disposition caractéristique de l'invention, l'appareil comporte une pièce à main, pour diriger un rayon laser sur un site d'intervention, par exemple constitué par un tissu physiologique ou une surface minéralisée, cette pièce à main comprenant un logement, une fibre optique conformée pour pouvoir être engagée dans ce logement avec une partie faisant saillie hors de ce dernier, des moyens permettant de fixer, de manière amovible, et de maintenir la fibre optique dans le logement, de manière qu'un rayonnement laser puisse être appliqué sur l'extrémité interne de la fibre optique et être émis par l'extrémité de la portion externe saillante de ladite fibre. Pour réaliser complètement les objectifs de l'invention, la pièce à main comprend un tube ou une structure comparable permettant d'acheminer et de diriger un jet ou un flux, d'un liquide de refroidissement qui, de préférence, peut être de l'eau, ou une solution de refroidissement et de nettoyage qui peut être employée en art dentaire, sur la zone sur laquelle l'émission laser est dirigée.

Bien entendu, d'autres objets de l'invention peuvent être obtenus par différentes combinaisons des moyens ou procédés décrits précédemment, et qui seront évidents pour les hommes de l'art à la lecture de la description détaillée qui suit.

Dans les dessins annexés :

– La figure 1 est une vue de côté, à caractère schématique et en coupe longitudinale, montrant les composants principaux d'un appareil construit pour réaliser différents traitements, selon l'invention.

– La figure 2 est une vue à caractère schématique, similaire à la figure 1, d'un deuxième appareil construit pour réaliser différents traitements selon l'invention.

On se reporte auxdits dessins pour décrire des exemples intéressants de mise en oeuvre du procédé et de réalisation du dispositif selon l'invention.

Suivant un premier aspect de la présente invention, une amélioration des actions de coupe des tissus minéralisés, est réalisée par l'utilisation d'un rayonnement laser polarisé ayant une polarisation P.

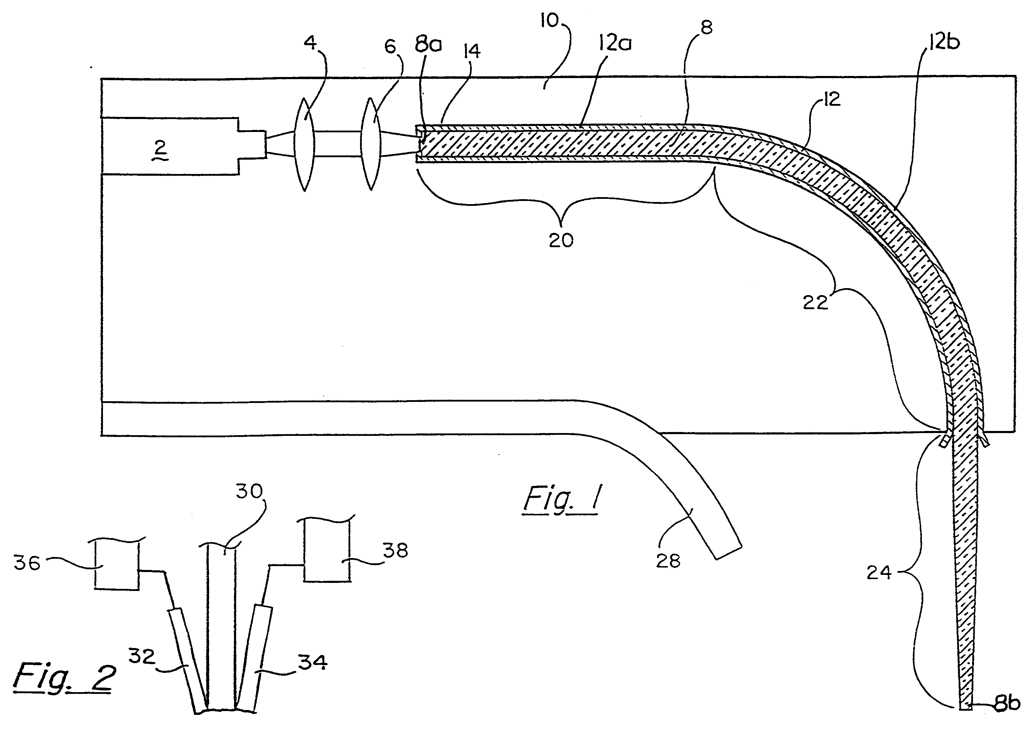

Un exemple de construction de dispositif permettant de produire un tel rayon polarisé, est montré à la figure 1.

Le rayonnement émis par un laser ND YAG désigné par la référence 2 est collimaté par une lentille convergente 4. Ensuite, un dispositif de polarisation linéaire, par exemple constitué par une lentille 6 de caractéristiques appropriées, réalise, à la fois, une polarisation linéaire du rayon collimaté et la convergence de ce rayon polarisé, jusqu'à l'extrémité d'entrée d'une fibre optique 8. Cette fibre 8 traverse la pièce à main 10 et sort de cette pièce à main 10 sur une longueur qui est nécessaire pour amener l'extrémité libre de la fibre 8 dans la région des tissus à traiter.

Comme une telle fibre optique peut subir une érosion à son extrémité distale après une période d'usage, il est préférable que cette fibre 8 soit installée dans la pièce à main 10 de façon qu'elle soit remplaçable d'une façon aisée et rapide. Le montage amovible de la fibre optique 8 est réalisé très simplement en munissant la pièce à main 10 d'un conduit de guidage et de maintien 12 dans lequel la fibre 8 peut être insérée et duquel elle peut être retirée, mais qui permet de maintenir la fibre engagée dans la pièce à main tout en positionnant de façon très précise son extrémité proximale en alignement avec la lentille 6. Comme le montre la figure 1, l'extrémité proximale du conduit peut être entaillée longitudinalement pour constituer des doigts 14 flexibles radialement vers l'intérieur et permettant de serrer l'extrémité proximale de la fibre 8. Comme cela est aussi montré à la figure 1, les extrémités proximales des doigts 14 peuvent être courbées radialement vers l'intérieur de manière à constituer des butées longitudinales pour l'extrémité proximale de la fibre 8. Cet agencement permet aussi à différents types de fibres, par exemple, des fibres présentant une certaine conicité, ou des fibres à l'extrémité cylindrique, d'être introduites dans une même pièce à main. La fibre 8 peut, par exemple, avoir une longueur totale de 5 cm, comprenant une portion proximale droite 20, d'une longueur de 1,5 cm, une portion intermédiaire courbe ou arquée 22 d'une longueur de 1,5 cm, et une portion distale terminale 24 ayant une longueur de 2 cm. A cet effet, le conduit de guidage et de maintien comporte une portion proximale rectiligne 12a disposée en alignement avec les lentilles 4 et 6, et une portion courbe 12b orientée vers la sortie de la pièce à main 10, ledit conduit de guidage et de maintien ayant une longueur plus réduite que celle des fibres optiques interchangeables utilisées.

La pièce à main 10 est complétée par un tube distributeur 28 servant à fournir un fluide liquide ou gazeux de refroidissement, généralement de l'eau, ce tube distributeur permettant de diriger un flux de fluide de refroidissement sur la région qui reçoit le rayonnement laser.

Différents traitements peuvent être réalisés utilisant cette émission laser pulsée avec un pic d'énergie de 1 à 600 mJ, un taux de répétition de 1 Hz à 1 kHz et, de préférence, de 50 Hz, une durée de pulse de 0,4 à 1 ms et un taux moyen d'énergie de l'ordre de 150 à 300 mJ. Pour les opérations de vaporisation ou de coupe, le rayonnement laser doit être focalisé sur une tache de petit diamètre et doit être émis sous forme de pulses et simultanément, un refroidissement doit être opéré de préférence au moyen d'un flux liquide ou d'un mélange liquide et air. D'autres types d'opérations peuvent être réalisés de préférence avec un faisceau ou rayon défocalisé, lorsque l'émission sous forme pulsée et/ou lorsque le refroidissement continu ne sont pas essentiels.

Suivant une caractéristique de l'invention, l'émission ou rayonnement laser est appliquée sous une forme polarisée linéaire avec une polarisation P parallèle à la surface qui doit être traitée ou aux cristaux minéraux qui doivent absorber l'énergie. Lorsque ce type de polarisation est employé, la reflectivité effective ou pouvoir de réflexion de la surface recevant la radiation ou rayonnement, varie en fonction de l'angle d'incidence que forme le rayonnement avec la surface à traiter. D'une façon spécifique, la réflectivité décroît quant l'angle d'incidence augmente jusqu'à un angle d'incidence légèrement inférieur à 90 degrés ; quant la réflectivité décroît, la proportion de radiation absorbée par le tissu augmente et favorise la vaporisation et l'effet de coupe. Ainsi, en donnant une orientation convenable au faisceau incident, obtenue par l'orientation adéquate de l'extrémité externe de la fibre 8, il est possible de contrôler la vaporisation ou l'action de coupe, d'une façon plus fine et plus efficace.

Suivant une autre particularité intéressante de l'invention, la fibre 8 par l'intermédiaire de laquelle la radiation est émise a une forme effilée ou conique dans la région ou l'émission est effectuée, c'est-à-dire dans la région de l'extrémité externe ou distale de ladite fibre. Il a été découvert que si une conicité convenable est donnée à la fibre, le rayonnement laser est émis tout au long de la zone effilée de la fibre, et peut donc être appliqué sur une portion plus étendue de la zone à traiter. Ceci est particulièrement avantageux pour réaliser des opérations d'élargissement ou de conformation de canaux dentaires. Sans une telle conicité, le faisceau laser est concentré à l'extrémité externe de la fibre 8. La concentration du faisceau à l'extrémité de la fibre est désirable pour d'autres procédures décrites dans le présent exposé et pour lesquelles, il n'est pas nécessaire d'utiliser une fibre à extrémité conique, mais pour réaliser l'évidement ou l'élargissement d'un canal, une telle concentration a tendance à produire des encoches ou des escaliers dans la paroi du canal, empêchant la préparation correcte de celui-ci. Par conséquent, il est préférable d'utiliser une fibre à extrémité distale effilée pour la procédure d'élargissement des canaux et pour d'autres procédures qui seront décrites dans la suite du présent exposé et dans lesquelles l'extrémité distale de la fibre doit être introduite à l'intérieur des canaux dentaires, et une fibre à extrémité distale cylindrique pour d'autres procédures décrites ci-après.

La longueur de la région effilée ou conique et le degré de conicité peuvent varier pour obtenir des émissions laser présentant les caractéristiques recherchées, en fonction des procédures à réaliser. Par exemple, une fibre 8 pour réaliser une opération particulière peut avoir une longueur de travail, par exemple constituée par sa portion distale 24 disposée à l'extérieur de la pièce à main 10 et correspondant à la longueur d'une lime mécanique qui est couramment utilisée pour la préparation mécanique d'un canal et une partie au moins de cette portion distale est effilée conformément à l'invention. Il est avantageux, pour une telle utilisation, que l'extrémité distale de la fibre 8 ait une conicité en fonction de la conicité de la lime mécanique qui a été utilisée pour la préparation mécanique du canal antérieurement à la préparation laser. A titre d'exemple, la portion distale 24 peut avoir avantageusement une longueur de 20 mm et peut avoir une partie effilée ayant une longueur de 16 à 20 mm partant du diamètre le plus petit de 200 μm pour donner une conicité allant de 600 à 200 μm sur une longueur de 16 à 20 mm. De préférence, le degré de conicité de la portion effilée de la fibre 8, est tel que le diamètre de ladite portion varie de 25 μm par millimètre de longueur de fibre. Une telle conicité doit être linéaire.

Toutefois, d'autres types de conicité plus ou moins grandes peuvent s'avérer intéressantes pour certaines utilisations particulières.

Pour ce type de procédure, c'est-à-dire pour l'utilisation d'un faisceau laser à l'intérieur de canaux, on peut utiliser un niveau d'énergie moyen de l'ordre de 150 à 250 mJ. La procédure d'élargissement du canal précédemment décrite peut être facilitée en colorant la paroi du canal avec une couleur noire ou sombre avant d'introduire la fibre à l'intérieur dudit canal. Ceci peut être réalisé au moyen d'une substance telle qu'un nitrate d'argent, introduite à l'intérieur du canal.

Par ailleurs, les dispositifs selon l'invention, peuvent avantageusement inclure la prévision d'un revêtement réfléchissant, par exemple le silice, sur la fibre optique en amont de sa portion distale effilée, pour confiner le rayonnement à l'intérieur de la fibre 8 jusqu'à ce que ledit rayonnement atteigne ladite portion distale effilée, c'est-à-dire jusqu'à l'extrémité d'émission de ladite fibre.

Suivant une importante caractéristique de l'invention, la fibre optique 8 qui peut être conique comme indiqué précédemment, peut être avantageusement réalisée dans un matériau ou dans une composition ayant un point de fusion bas, par exemple en verre ou en plastique, ou bien encore un produit compatible avec les tissus à traiter tel qu'un produit calcique comme la brushite ou le phosphate tricalcique ou hydroxyapatique, de telle sorte que l'énergie transmise par le rayonnement laser assure la fusion de la fibre 8 et permette ainsi le comblement ou le scellement d'une cavité sur la dent ou dans un os, tout en permettant à ces produits de se fixer et de se fondre sur les parois de ladite cavité.

Une telle opération peut être réalisée pour l'obturation d'un canal dentaire qui est la dernière opération de la procédure endodontique. Cette opération peut être réalisée de manière à fermer ou à sceller complètement un canal dentaire dans la région du foramen, ou apex. Cependant, cette opération peut aussi être effectuée pour remplir tout type de cavité dans la dent, c'est-à-dire dans l'émail ou dans la dentine, ou dans la matière osseuse.

De manière préférée, le point de fusion de tels matériaux ne doit pas être supérieur à 600 degrés. Au-dessus de cette température, il existe des risques d'effets thermiques entrainant des dommages ou des traumas dans les tissus environnants. Lorsque la fibre 8 est composée essentiellement de silice, elle est parfaitement compatible avec l'émail, la dentine et l'os. Le plastique est utilisé seulement pour bénéficier d'une température de fusion basse. Cependant, toute matière plastique compatible avec les tissus physiologiques concernés peut être utilisée. De façon pratique, la composition ne devra pas avoir un point de fusion plus bas que 100 degrés.

Certaines fibres optiques ayant un point de fusion à 150 degrés et au-dessus sont commercialisées par les Sociétés Fiberguide ou General Fiber Optics, toutes deux situées dans l'Etat de New Jersey (U.S.A.).

Avant d'introduire la fibre dans le canal dentaire appelé à être fermé hermétiquement, les parois canalaires peuvent avantageusement être colorées avec une couleur noire, par exemple, avec du nitrate d'argent pour augmenter la transmission de l'énergie sur les lesdites parois canalaires.

Les bénéfices procurés par une fibre 8 à point de fusion bas, peuvent être accrus en la dotant d'un revêtement, dans sa portion qui doit être fondue, ce revêtement ayant une composition lui permettant d'être fondu par l'émission ou rayonnement laser en constituant une partie du matériau d'obturation. Les ingrédients de ce revêtement peuvent être sélectionnés en fonction d'une variété de buts à atteindre. Par exemple, le revêtement peut contenir un ou plus des produits suivants : - un matériel sombre, tels le carbone végétal ou animal de couleur noire qui absorbe largement l'émission ou rayonnement laser, et qui favorise ainsi la fusion et la fonte du revêtement en réponse à l'application d'une émission ou rayonnement ayant un bas niveau d'énergie ; - une poudre de phosphate de calcium, par exemple d'hydroxyapatite qui est un des composants naturels des tissus minéralisés corporels, incluant l'os, l'émail, la dentine, le cément, et qui peut servir ainsi de produit d'obturation permettant d'améliorer l'acceptation biologique ou tolérance physique de la composition qui peut être à base de verre ou de plastique ; - une poudre de céramique dont la fonction est essentiellement celle d'un produit d'obturation inerte ;
- et/ou tout autre composition compatible avec les tissus qui doivent être comblés.

Suivant l'état de la technique, de tels matériaux ont déjà été employés pour remplir des cavités dentaires ; toutefois, ces produits sont comprimés mécaniquement à l'intérieur de la cavité, quelquefois en utilisant un produit de liaison. En remplissant une cavité avec un corps de verre fondu ou avec une matrice de verre fondu contenant différentes combinaisons des ingrédients ou composants ci-dessus, selon l'invention, on obtient un meilleur remplissage de la cavité et une meilleure adhérence du produit de remplissage aux parois de ladite cavité, ou meilleure coalescence des matières en conctact.

Pour permettre à la fibre 8 d'atteindre la température de fusion, il suffira d'émettre des radiations à un niveau moyen d'énergie compris entre 50 et 200 mJ, la radiation pouvant être, soit continue, soit pulsée. L'épaisseur du revêtement peut correspondre jusqu'à la moitié du diamètre de la fibre 8. Pour améliorer le contact du matériau de comblement avec les parois canalaires, la fibre 8, qu'elle soit avec revêtement ou sans revêtement, doit être légèrement poussée d'une façon longitudinale, le long du canal, en permettant ainsi de lui communiquer une certaine compression pendant que la radiation est émise. Une cavité ou une ouverture dans l'os ou dans la dent, peut être aussi remplie en préparant une pâte composée d'une poudre contenant un ou plus des ingrédients qui ont été décrits précédemment et un liquide tel que l'acide phosphorique $H_3PO_4$ qui pourra agir avec un des ingrédients de la mixture, par exemple l'hydroxyapatite, en appliquant cette pâte dans la région qui doit être remplie ou complètement scellée, et en appliquant le rayonnement laser à la pâte. Dans ce cas, également, une substance noire pourra être incluse dans la pâte pour augmenter sa capacité d'absorption de l'énergie laser.

Suivant une autre disposition caractéristique de l'invention, une obturation ou revêtement de cavité ou paroi de tissu minéralisé peut être réalisé en projetant une mixture ou mélange des différents ingrédients des types qui ont déjà été mentionnés précédemment, dans la cavité ou dans l'évidement qui doit être rempli ou sur la surface qui doit être revêtue, en même temps que l'on applique un rayonnement laser sur le mélange projeté, soit par l'intermédiaire d'une fibre optique, soit sans fibre optique, de façon à engendrer une réaction photochimique entraînant la formation d'une masse d'obturation dure, non poreuse et résistante qui s'adapte et se fond sur les tissus physiologiques auxquels elle se trouve solidement fixée ou soudée. Les tissus sur lesquels sont projetés les produits d'obturation ou de revêtement pendant l'application de rayonnement laser, sont l'émail, la dentine, l'or ou l'os.

Lorsqu'on réalise des obturations ou des revêtements au moyen de ce procédé, ces ingrédients qui doivent réagir peuvent être projetés ensemble lorsqu'ils n'ont pas la possibilité, de réagir en l'absence de radiation laser. Par contre, lorsque les composants peuvent réagir en l'absence de radiation laser, ils sont projetés à partir de deux sources séparées de façon à se mélanger et à se trouver exposés au rayonnement laser, durant le trajet des particules solides et/ou des particules liquides projetées en direction de la cible ou site d'intervention.

De manière préférée, le système de projection des produits d'obturation ou de revêtement est réalisé de façon que la réaction photochimique commence pendant que les ingrédients sont en vol et s'achève après que lesdits ingrédients soient entrés en contact avec la surface de la cible.

Les composants principaux du dispositif permettant la mise en oeuvre d'un tel procédé, sont montrés à la figure 2. Ce dispositif comprend une fibre optique 30 pour permettre d'acheminer le faisceau laser jusqu'à la région qui doit être obturée ou revêtue du produit d'obturation ou de revêtement, et deux tubes d'alimentation et de projection 32 et 34 qui sont raccordés à des réservoirs qui contiennent les composants du matériau d'obturation ou de revêtement, lesquels sont désignés par les références 36 et 38.

Pour citer un exemple d'application du procédé qui peut être mis en oeuvre à l'aide de l'arrangement illustré schématiquement à la figure 2, une mixture ou mélange des matériaux ou ingrédients précédemment cités, tels que, hydroxyapatite, céramique et un matériau de couleur sombre, par exemple, du carbone noir, tous sous forme de poudres, est projeté par l'intermédiaire du tube 32, ces projections étant effectuées à l'aide d'un flot ou courant d'air émis dans ledit tube, pendant que l'acide phosphorique ($H_3PO_4$) est projeté par l'intermédiaire du tube 34, au moyen d'un deuxième flot ou courant d'air. Les deux flots ou courants sortant des tubes 32 et 34 et transportant les ingrédients susmentionnés, se rencontrent et se mélangent dans une région qui est irradiée par le rayonnement laser émanant de la fibre 30. Il en résulte une réaction photo-chimique permettant d'obtenir du phosphate de calcium. En fonction des ingrédients du mélange, la réaction pourra produire du phosphate de calcium monobasique, $Ca(H_2PO_4)_2.H_2O$, ou du phosphate de calcium bibasique $CaHPO_4.2H_2O$, ou du phosphate de calcium tribasique $CaO(OH)_2(PO_4)_6$. Pour la mise en oeuvre de cette procédure, l'émission au rayonnement laser est, de préférence, défocalisée pour couvrir toute la région d'irradiation dans laquelle l'obturation ou le revêtement doit être réalisé. L'émission laser peut être soit continue, soit pulsée, et, dans ce cas, la projection d'un liquide de refroidissement n'est pas nécessaire étant donné que l'on a, par ailleurs, une émission d'air qui amène les particules.

Suivant une autre disposition caractéristique de l'invention, la faculté procurée par un laser ND YAG, de vaporiser les matières sombres est utilisée pour détruire d'une façon sélective les bactéries qui peuvent être présentes sur les dents ou sur les gencives et qui, si elles ne sont pas inquiétées, sont la cause de caries ou d'infections. Suivant l'invention, les bactéries qui doivent être éliminées, sont colorées en noir ou en couleur sombre, au moyen d'un colorant sélectif et sont ensuite exposées à un rayonnement laser ayant une énergie relativement faible qui est suffisante pour détruire les bactéries. A titre d'exemple non limitatif, les bactéries peuvent être colorées au moyen de bleu de méthylène, de "dye agaroses", de bleu tripane, de nigrosine, de naphtol bleu ou noir. La nigrosine permet de colorer les bactéries en noir. Ces colorants peuvent être utilisées pour colorer, entre autres, les staphylocoques, les streptocoques, les veillonella flora et les bactéroïdes mélanogenicus.

Le résultat de cette coloration est que ces bactéries pourront être détruites par l'application d'une émission ou rayonnement laser à très bas niveau d'énergie. Par exemple, ce résultat peut être obtenu en appliquant une émission ou rayonnement laser à un niveau d'énergie compris entre 2 et 10 mJ, sous forme d'un faisceau laser pulsé, avec un taux de répétition de 50 Hz et des durées de pulses de 0,8 ms, pour un temps d'application de l'ordre de 3 à 4 secondes. Quand ces paramètres sont établis, la destruction, c'est-à-dire, vaporisation de la bactérie est accomplie de telle sorte que les tissus environnant ne sont pas affectés ; on obtient donc une destruction sélective des bactéries.

Sans une telle coloration, l'obtention d'un résultat similaire exigerait une énergie de l'ordre de 100 mJ, c'est-à-dire un niveau d'énergie au moins dix fois supérieur.

Comme indiqué ci-dessus, les procédures selon la présente invention peuvent être utilisées pour obturer ou réaliser un revêtement sur la matière osseuse. L'os ayant une composition qui est presque similaire à celle des tissus de la dent, les procédures d'obturation ou de revêtement décrites précédemment peuvent être mises en oeuvre de la même façon. par exemple, un remplissage ou un revêtement peut être réalisé en appliquant un mélange de poudres d'hydroxyapatite et de céramique ainsi que de l'acide phosphorique, et en soumettant ce mélange à une radiation laser pour produire un effet photochimique permettant d'obtenir une masse fondue composée d'une forme de phosphate de calcium. Un agent d'assombrissement ou de coloration tel qu'indiqué précédemment, peut être additionné à l'un et/ou l'autre des composants, pour réduire le niveau d'énergie nécessaire pour effectuer la fusion. L'addition d'un tel matériau noir ou sombre permet d'utiliser un rayonnement laser dont le niveau d'énergie peut ne pas être supérieur à 200 mJ.

Il est aussi possible d'inclure dans le mélange de poudres, 50 à 60 % d'os naturel, le reste des constituants dudit mélange pouvant être de l'hydroxyapatite ou de la céramique. Les composants susmentionnés peuvent être appliqués sous forme de pâte ou bien peuvent être projetés au moyen d'un système du genre de celui qui est illustré à la figure 2.

Afin de permettre de couper, de façon satisfaisante, la matière osseuse, avec une émission ou rayonnement laser, il est important d'appliquer simultanément sur la zone irradiée un fluide de refroidissement, liquide ou gazeux, qui peut être de l'eau ou de l'air, ou un mélange d'air et d'eau, pour éviter une carbonisation de la matière osseuse. L'eau, comme cela a déjà été indiqué, n'absorbe pas l'énergie du rayonnement laser à la longueur d'onde de 1,06 µm, mais permet d'éliminer la chaleur qui est absorbée par l'os de sorte que l'on évite la carbonisation.

Pour la mise en oeuvre des procédures précédemment décrites, on peut utiliser, l'émission ou rayonnement d'autres types de laser, si ce rayonnement a une longueur d'onde qui est absorbée d'une façon importante par l'hydroxyapatite ou par les tissus minéralisés, mais qui n'est pas absorbée de façon significative par l'eau. Dans ce cas, on peut, par exemple, utiliser des lasers Holmium, ou des lasers Excimer, ou des lasers Saphir-Titane. Le laser Saphir Titane offre l'avantage d'être accordable, de sorte qu'un unique laser peut être accordé, par exemple, pour émettre un rayonnement, soit à une longueur d'onde aux environs de 760 nm qui s'est avérée la mieux appropriée pour couper les tissus physiologiques mous, soit à une longueur d'onde voisine de 1 µm qui s'est avérée la plus intéressante pour couper des tissus durs ou des tissus minéralisés tels que l'os ou les tissus physiologiques minéralisés.

Suivant une autre disposition caractéristique de l'invention, un rayonnement laser focalisé, ayant une longueur d'onde, un niveau d'énergie, une durée de pulse, et un taux de répétition de pulse tel que décrite précédemment, peut être utilisé pour détruire, c'est-à-dire vaporiser, le tartre et le calcul, particulièrement autour de la racine d'une dent, y compris dans une poche parodontale sous la gencive. En même temps, le rayonnement agira de façon à vaporiser le cément nécrotique dans la région traitée, à stériliser le cément qui reste, et à permettre la fusion de la dentine qui se trouve au-dessous du cément, en obtenant ainsi la fermeture des tubules ou canalicules dentinaires dans la dentine et, de la sorte, une réduction de l'hypersensibilité de cette région de la dent. Pour ces opérations, le taux d'énergie préféré se situe entre 200 et 300 mJ. Pendant l'application du rayonnement, un fluide de refroidissement liquide ou gazeux est projeté sur le site d'intervention, pour protéger les tissus dentaires de la carbonisation.

**Revendications**

1. - Procédé utilisant la radiation laser pour interventions sur des tissus minéralisés divers, notamment pour la coupe de tissus minéralisés tels que, par exemple, les tissus physiologiques minéralisés incluant l'émail dentaire, la dentine et l'os, caractérisé en ce qu'il comprend la production d'un rayonnement laser à une longueur d'onde qui est absorbée de façon plus importante par l'hydroxyapatite que par l'eau, la polarisation linéaire de ce rayonnement, la focalisation de ce rayonnement polarisé en un rayon de diamètre particulièrement petit, et l'application du faisceau polarisé sur la surface du tissu qui doit être coupée de façon que le rayon ait un angle d'incidence sélectionné par rapport à ladite surface ; procédé suivant lequel le rayonnement est polarisé et le rayon polarisé est dirigé de sorte que le rayonnement frappant la surface ait une polarisation parallèle à ladite surface ; un flot de fluide de refroidissement liquide ou gazeux, est, de préférence, dirigé sur les tissus dans la zone d'application du rayon laser, pour éviter la carbonisation de ces derniers.

2. - Procédé utilisant la radiation laser pour interventions sur des tissus minéralisés divers, notamment pour l'obturation ou le revêtement de tissus minéralisés, tels que, par exemple, les tissus physiologiques incluant, l'émail dentaire, la dentine et l'os, avec un produit à base de calcium apte à se fixer sur la surface desdits tissus, caractérisé en ce qu'il comprend :
   – l'utilisation d'au moins deux composants qui lorsqu'ils sont mélangés et exposés à un rayonnement laser à une longueur d'onde déterminée produisent une réaction photochimique entraînant la formation dudit produit à base de calcium ;
   – la projection de ces composants sur la surface ;
   – et l'application, sur ces derniers, d'un rayonnement laser à une longueur d'onde sélectionnée, cette application du rayonnement laser sur les composants s'effectuant au moins partiellement avant que lesdits composants n'atteignent la surface traitée, de façon à engendrer une réaction photochimique et à assurer la fixation ou soudure du produit contenant du calcium résultant de cette interraction, sur le tissu traité.

3. - Procédé utilisant la radiation laser pour interventions sur des tissus minéralisés divers, notamment pour remplir un canal dentaire de façon à permettre de sceller ce canal, comprenant :

– l'utilisation d'une fibre optique contenant de la silice, cette fibre optique ayant une température de fusion très basse et étant dimensionnée pour être introduite à l'intérieur du canal ;

– l'introduction de cette fibre dans le canal, de manière qu'elle s'étende le long de la région qui doit être remplie ;

– et l'acheminement à travers la fibre optique d'un rayonnement laser d'une longueur d'onde et d'un niveau d'énergie suffisants pour permettre la fusion de la portion de la fibre qui se trouve à l'intérieur du canal et assurer ainsi la soudure de la silice contenue dans la fibre, sur les parois canalaires.

**4.** - Procédé selon la revendication 3, comprenant, avant l'insertion de la fibre, le revêtement de la portion de la fibre optique qui doit être insérée à l'intérieur du canal avec une composition apte à être fondue et fixée ou soudée sur les parois canalaires.

**5.** - Procédé utilisant la radiation laser pour interventions sur des tissus minéralisés, notamment pour détruire les bactéries présentes sur les tissus physiologiques, dans la bouche, caractérisé en ce qu'il comprend : la coloration des bactéries de façon à leur donner une couleur sombre ; et l'exposition des bactéries ainsi colorés à un rayonnement laser à une longueur d'onde et à un niveau d'énergie suffisants pour détruire les bactéries ; le niveau d'énergie du rayonnement laser étant sélectionné de telle sorte que les tissus physiologiques sur lesquels les bactéries sont présentes, ne sont pas affectés par le rayonnement laser appliqué.

**6.** - Procédé utilisant la radiation laser pour interventions sur des tissus minéralisés divers, notamment pour vaporiser le tartre et les calculs autour de la dent ou de la racine dentaire, caractérisé en ce qu'il comprend la production d'un rayonnement laser à une longueur d'onde qui est absorbée plus fortement par l'hydroxyapatite que par l'eau ; et l'application du rayonnement à l'emplacement du tartre ou du calcul adjacent à la racine dentaire, pendant une durée suffisante pour vaporiser le tartre et le calcul, le rayonnement ayant, de préférence, des pics d'énergie de l'ordre de 200 à 300 mJ.

**7.** - Appareil utilisant la radiation laser pour interventions sur des tissus minéralisés divers, tels que, par exemple, des tissus physiologiques minéralisés incluant, en particulier, l'os et les différents tissus dentaires, cet appareil comprenant une source d'émission (2) d'un rayonnement laser et une fibre optique (8) ayant une extrémité proximale (8a) disposée pour recevoir le rayonnement émis par la source laser (2) et une extrémité distale (8b) dimensionnée pour pouvoir être introduite dans un canal dentaire ou autre, caractérisé en ce qu'un dispositif (6) de polarisation linéaire est intercalé entre ladite source laser (2) et ladite extrémité proximale (8a) de la fibre optique (8), ce dispositif (6) permettant une polarisation linéaire du faisceau ou rayonnement émis par la source laser.

**8.** - Appareil selon la revendication 7, caractérisé en ce qu'une lentille convergente (4) est disposée entre la source laser et le dispositif de polarisation linéaire (6), cette lentille permettant de collimater le rayonnement émis par la source laser avant son passage dans ledit dispositif.

**9.** - Appareil utilisant la radiation laser pour interventions sur des tissus minéralisés divers, notamment pour enlever des tissus physiologiques d'un canal dentaire ou de tout autre canal, par application sur le tissu d'un rayonnement laser, ce dispositif incluant une source d'un tel rayonnement laser (2) et une fibre optique (8) ayant une extrémité proximale (8a) disposée pour recevoir le rayonnement émis par ladite source et une extrémité distale (8b) dimensionnée pour pouvoir être introduite à l'intérieur d'un canal dentaire ou autre, caractérisé en ce que cette fibre optique (8) est conique ou effilée dans sa portion voisine de son extrémité distale (8b), de sorte que le diamètre de cette portion décroît en direction de ladite extrémité distale de la fibre ; de préférence, la conicité de la fibre est linéaire, et le degré de conicité de sa portion effilée est tel que le diamètre de ladite portion varie de 25 µm par millimètre de longueur de fibre.

**10.** - Appareil utilisant la radiation laser pour interventions sur des tissus minéralisés divers, notamment pour remplir un canal dentaire ou autre, comprenant une source (2) de rayonnement ou radiation laser, et des fibres optiques interchangeables (8) ayant une extrémité proximale disposée pour recevoir l'émission laser et une extrémité distale dimensionnée pour être insérée à l'intérieur d'un canal, caractérisé en ce que ces fibres (8) contiennent, au moins dans leur portion voisine de leur extrémité distale (8b) dimensionnée pour pouvoir être introduite dans un canal, un matériau ayant une température de fusion basse, compatible avec les tissus, et apte à se fixer ou à se souder sur ces derniers par fusion sous l'effet d'une radiation laser.

**11.** - Appareil selon la revendication 10, caractérisé en ce que les fibres interchangeables (8) comprennent, dans leur portion voisine de leur extrémité distale (8b) dimensionnée pour pouvoir être introduite dans un canal, un revêtement d'un matériau ayant une température de fusion basse, compatible avec les tissus, et apte à se fixer ou à se souder sur les tissus minéralisés par fusion sous l'effet d'une radiation laser.

**12.** - Appareil suivant la revendication 11, caractérisé en ce que le revêtement de la portion proche de l'extrémité distale des fibres interchangeables contient une matière sombre, telle que le carbone végétal ou animal.

**13.** - Appareil selon l'une des revendications 11 ou 12, caractérisé en ce que le revêtement contient de l'hydroxyapatite.

**14.** - Appareil selon l'une quelconque des revendications 7 à 13, caractérisé en ce qu'il comporte un conduit de guidage et de maintien (12) pour le montage amovible de la fibre optique (8), ce conduit comprenant une portion rectiligne (12a) disposée en alignement avec les dispositifs (4, 6) permettant de collimater et de polariser linéairement le rayonnement laser, et une portion courbe (12b) orientée vers la sortie de la pièce à main (10), ledit conduit de guidage et de maintien ayant une longueur plus réduite que celle des fibres optiques amovibles utilisées et son extrémité proximale est, de préférence, agencée pour servir de butée à la fibre.

**15.** - Appareil utilisant l'effet laser pour interventions sur des tissus minéralisés divers, notamment pour obturer une cavité ou pour déposer un revêtement sur un tissu minéralisé, comprenant une source (30) d'émission d'une radiation laser, caractérisé en ce qu'il comprend au moins deux dispositifs de projection (32, 34) permettant de projeter simultanément, en direction du site d'intervention, les ingrédients destinés à constituer le matériau d'obturation ou de revêtement par réaction photochimique sous l'effet de l'émission d'une radiation laser.

**16.** - Appareil selon l'une quelconque des revendications 7 à 14, caractérisé en ce que les fibres optiques interchangeables (8) comportent, en amont de leur portion distale effilée, un revêtement réfléchissant.

_Fig. 1_

_Fig. 2_

EP 0 487 435 A2